# EUROPEAN PATENT APPLICATION

(11) **EP 4 763 214 A1**
(43) Date of publication of application: **24.06.2026**
(21) Application number: 24221750.3
(22) Date of filing: 19.12.2024
(51) Int. Cl.: A61K 36/28, A61K 36/575, A61K 31/00, A61K 31/05, A61K 31/164, A61P 3/06

(54) **HERBAL COMPOSITION FOR ACTIVATION OF THE ENDOCANNABINOID SYSTEM**

(71) Applicant: Bayer Consumer Care AG, 4052 Basel (CH)
(72) Inventor: Ammar, Ramy Mohammed Ahmed, 64319 Pfungstadt (DE); Bedal, Konstanze Barbara, 64285 Darmstadt (DE)
(74) Representative: BIP Patents

(57) **Abstract**

The present invention relates to herbal compositions and methods to activate the endocannabinoid system in order to alleviate stress and its attendant adverse health effects.

## Description

The present invention relates to herbal compositions and methods to activate the endocannabinoid system in order to alleviate stress and its attendant adverse health effects.

Mental wellness needs are on the rise, especially with the surge during the pandemic. Stress is at the core of various mental health problems. As consumers take more interest in mental wellbeing, many are looking to reduce stress and improve sleep as these are considered key pillars for holistic health. This provides an opportunity to provide holistic solutions with multi-benefit mental support, for instance, by the modulation of the body's own endocannabinoid system.

The endocannabinoid system (ECS) is one of the most crucial systems in the human organism, exhibiting multi-purpose regulatory character. It is engaged in a vast array of physiological processes, for example mood regulation, cognitive functions, neurogenesis and neuroprotection. The components (proteins) of the ECS include receptors, their ligands, and enzymes responsible for their biosynthesis and degradation/deactivation. The ECS has increasingly become a favourable target for the treatment of various diseases as many of its components are distributed widely throughout the body and take part in cell-signaling pathways involved in the pathophysiology of many types of diseases.

A variety of methods are known for stimulating the ECS, including ingestion of Omega-3 fatty acids, of certain terpenes, of certain herbs and teas, of dark chocolate, and particularly of cannabinoids.

Cannabinoids are chemicals in cannabis (and many other plants) that interact with the ECS. The most prominent cannabinoids are tetrahydrocannabinol (THC) and cannabidiol (CBD). The human body produces molecules similar to THC/CBD, called endocannabinoids. The two most studied endocannabinoids in the body are, 2-arachidonoylglycerol (2AG) and anandamide (N-arachidonoylethanolamine) along with their receptors, cannabinoid type 1 and type 2 receptors; CB1 and CB2.

Cannabinoid modulation to the ECS influences numerous physiological processes, including stress, anxiety, appetite, immune response, pain, movement, and memory. In the Relax and Restore space, evidence for the role of ECS is highest for stress and anxiety which could have a cascading effect on sleep.

Despite a controversial recent change in German legislation, many well-known issues exist with the intake of THC for medicinal or other purposes.

The European Commission considers that CBD qualifies as a novel food provided it meets the conditions of EU legislation on novel foods. Early 2022, following the submission of numerous applications for CBD under the novel food regulation, the Commission asked EFSA to provide its opinion on whether CBD consumption is safe for humans; EFSA's scientists could not establish the safety of CBD as a novel food due to data gaps and uncertainties about potential hazards related to CBD intake.

Thus, there exists a strong need for herbal compositions and methods to activate the endocannabinoid system that do not rely on THC and/or CBD to provide positive effects.

More specifically, it would be highly desirable to find a herbal composition that increases the release of ideally both Anandamide and 2-2-Arachidonylglycerol without THC or CBD.

Such herbal extracts could act as cannabimimetics and modulate the ECS, as an alternative to THC or CBD. Cannabimimetics are molecules beyond THC/CBD but are chemically similar.

It was now surprisingly found that the above technical objective could be met by a composition comprising comprising extract of Echinacea purpurea L and extract of Mangnolia officinalis.

Such a combination was found to show a synergistic effect in the release of Anandamide.

The extract of Echinacea purpurea L, commonly known as purple coneflower, is derived from the flowering plant native to North America. It is widely recognized for its potential immune-boosting properties and has been used traditionally to alleviate symptoms of the common cold and other respiratory infections. The extract contains a variety of bioactive compounds, including alkamides, caffeic acid derivatives, and polysaccharides, which are believed to contribute to its immunomodulatory effects.

The extraction of Echinacea purpurea L may typically be done by several known methods, such as Maceration, Percolation, Supercritical Fluid Extraction and Soxhlet Extraction.

These methods are employed to isolate the bioactive components from Echinacea purpurea L, yielding extracts that can be used in various forms, including tinctures, powders, and liquid preparations for medicinal and dietary supplement purposes.

The extract of Magnolia officinalis, commonly known as magnolia bark, is derived from the bark of the Magnolia officinalis tree, which is native to China. This extract is known for its potential therapeutic properties, including anti-anxiety, anti-inflammatory, and antioxidant effects. The bioactive compounds in the extract, such as honokiol and magnolol, are believed to contribute to its pharmacological activities. Magnolia officinalis extract has been used in traditional medicine for its anxiolytic and calming effects.

The extraction of Magnolia officinalis may typically be effected the same known methods as mentioned for Echinacea purpurea L above.

The content of the extracts in the inventive composition is not particularly limited.

In one embodiment, the content of extract of Echinacea purpurea L is in the range from 1 % by weight to 50 % by weight based on the total weight of the composition. In another embodiment, the concentration of extract of Echinaceae purpurea L is in the range from 3 % by weight to 10 % by weight based on the total weight of the composition.

In one embodiment, the content of extract of Mangnolia officinalis is in the range from 1 % by weight to 50 % by weight based on the total weight of the composition. In another embodiment, the concentration of extract of Mangnolia officinalis is in the range from 2 % by weight to 10 % by weight based on the total weight of the composition.

In another embodiment the composition additionally comprises Palmitoylethanolamide. Palmitoylethanolamide (PEA) is a fatty acid amide naturally produced within the human body. It is also found in various foods such as egg yolk, peanuts, and soybeans. PEA is known for its anti-inflammatory and pain-relieving properties, and it has been studied for its potential therapeutic effects in conditions such as chronic pain, inflammation, and neurological disorders. Additionally, PEA has been shown to interact with the endocannabinoid system.

It was surprisingly found that a composition comprising extract of Echinaceae purpurea L, extract of Mangnolia officinalis and Palmitoylethanolamide shown a synergistic effect in the release of both Anandamide and 2-2-Arachidonylglycerol.

In one embodiment, the content of Palmitoylethanolamide is in the range from 1 % by weight to 50 % by weight based on the total weight of the composition. In another embodiment, the concentration of Palmitoylethanolamide is in the range from 3 % by weight to 10 % by weight based on the total weight of the composition.

The inventive composition may additionally comprise one or more usual excipients, such as:
- Fillers and Binders: Examples include lactose, cellulose, and starch, which are used to help in the formation and binding of tablets or capsules.
- Disintegrants: Substances such as croscarmellose sodium or cross-linked PVP aid in the breakdown of tablets in the digestive tract for better absorption.
- Coatings: These include substances like shellac or hydroxypropyl methylcellulose, which are used to coat tablets for protection or to control their release.
- Solvents: Ethanol, glycerin, and propylene glycol are commonly used as solvents in liquid herbal extracts and tinctures.
- Emulsifiers: These help stabilize emulsions and include substances like lecithin or polysorbates, often used in topical preparations.
- Preservatives: For liquid formulations, preservatives like benzyl alcohol or potassium sorbate may be used to prevent microbial growth.
- Flavorings and Sweeteners: In oral liquids or teas, natural or artificial flavorings and sweeteners are often added to improve taste and palatability.
- Emollients and Base Materials: For topical preparations, base materials such as petroleum jelly, lanolin, or beeswax may be used as emollients or vehicle components.

The inventive composition may be formulated in a number of ways, which are known to the skilled person.

In one embodiment, the inventive composition may be formulated into Capsules (including soft gel capsules) or Tablets. These dosage forms provide accurate dosing and ease of administration by convenient oral consumption.

In another embodiment, the inventive composition may be formulated into Tinctures or Liquid Extracts, often using alcohol or glycerin as a solvent. These forms allow for easy dosing and absorption.

In another embodiment, the inventive composition may be formulated into Topical Preparations, such as creams, ointments, and gels for topical application. These formulations are used for skin conditions, muscle pain, and other localized issues.

In yet another embodiment, the inventive composition may be formulated into Teas and Infusions, allowing for oral consumption in a liquid form. This method is often used for herbal remedies that require infusion in hot water.

In yet another embodiment, the inventive composition may be formulated into Sprays and Inhalants for respiratory or nasal application, providing localized relief.

Another aspect of the present invention is the use of a composition comprising extract of Echinaceae purpurea L, extract of Mangnolia officinalis and Palmitoylethanolamide in a method of treatment, wherein the method of treatment comprises administering said composition to a person in need thereof.

### Examples

Human microglia cells were used in the present experiments. The human microglia clone 3 cell line HMC3 was used in this study and has been duly authenticated by the American Type Culture Collection (ATCC^{®}CRL-3304). These cells have retained the properties of primary microglial cells. According to ATCC they represent a convenient system for the biochemical analysis of the functions of microglial cells. Moreover, an extensive review suggested that this cell line should be regarded as a unique experimental model as these cells are capable of responding to a pattern of chemokines and inflammatory stimuli regulating the expression of typical activation markers of microglia (Russo et al. J Neuroinflammation 2018). As inflammatory stimulus lipopolysaccharides (LPS) were used that are described as agonist of the toll-like receptor-4 (TLR-4) responsible for the stimulation of the immune system (Cunningham C. 2013) and for activating microglia cells (Malek N et al. 2015).

The assessed plant extracts were selected based on previous in-vitro and in-vivo studies showing their impacts individually on different targets of the endocannabinoid system.

The aim of the experiments was to evaluate the impact of different combinations of plant extracts / drugs based on their influence on the enocannabinoid system and to assess the additive / supra-additive effect of this combination in nourishing the endocannabinoid system. Plant extracts of Echinaceae purpurea L (E) and Mangnolia officinalis (M) (in the following summarized as "plant extracts") as well as Palmitoylethanolamide (PEA), omega-3 fatty acids (Ω-3) and cannabidiol (CBD) as reference drugs (in the following summarized as "drugs") were investigated for their anti-inflammatory activity, their effect on the anandamide (AEA) and 2-Arachidonoylglycerol (2-AG) release on the amount of Fatty-Acid Amide Hydrolase (FAAH) as well as on the expression of genes as part of or relevant to the endocannabinoid system in the human microglia clone 3 (HMC3) cells.

### Materials and Methods

### Chemicals, Solutions, Assays

Chemicals and Solutions: PBS (Gibco), Miminum Essential medium according to Eagle with salts according to Earle (EMEM) (ROTI^{®}Cell Eagles MEM / Earles,Roth), 0,05%Trypsin/0,02% EDTA (Sigma), Heat inactivated foetal Calf Serum Gold (PAA); Lipopolysaccharides from Escherichia coli O111:84 (Sigma-Aldrich); CIS-4,7,10,13,16,19-Docosahexaenoicacid (Sigma-Aldrich), Palmitoylethanolamide (PEA), Resazurin Solution 0.1% (BIOTREND), Human IL-6 Elisa (DuoSet, Biotechne), Human Anandamide (AEA) ELISA Kit (abx258779, Abbexa), Human Fatty-Acid Amide Hydrolase 1 (FAAH) ELISA Kit (abx387238, Abbexa), Human 2-AG (2-Arachidonoylglycerol) ELISA Kit (MBS8807466, MyBioSource).

The plant extracts / drugs were provided by Steigerwald Arzneimittelwerk GmbH / Bayer Consumer Health. Stock solutions were prepared as indicated in Table 1 (STW) with DMSO or EMEM). Medium based solutions were sterile filtered. All solutions were aliquoted and kept frozen in -80C° and thawed directly before the experiment. Solutions were used initially in concentration ranges as shown in **Table 1.** Dilutions prepared in EMEM resulted in solutions with DMSO concentrations ≤ 0.1%. Five combinations were prepared. Combination 1 (**Comb1**) consisted of equal volumes of the Magnolia and the Echinaceae solution in the concentrations as provided in **Table 2.** Combination 2 (**Comb2**) consisted of equal volumes of Magnolia, Echinaceae and PEA. Combination 3 (**Comb3**) consisted of equal volumes of Magnolia, Echinacea, PEA and CBD, Combination 4 (**Comb4**) consisted of the two herbal extracts of M and E, PEA and -3, Combination 5 (**Comb5**) consisted of the 2 herbal extracts of M and E, PEA, -3 and CBD. Please note that for the viability assays the designation of Comb3 and Comb4 is reversed. The composition of the combinations for the experiments and the resulting concentrations in the solutions C1 to C5 are provided in **Table 2.**

**Table 1. Concentrations of single solutions used for treatment**

| **Compoun ds** | **C1** | **C2** | **C3** | **C4** | **C5** | **C6** |
|---|---|---|---|---|---|---|
| **CBD (µg/ml):** | 30.60 | 10.20 | 3.40 | 1.10 | 0.40 | 0.13 |
| **E (µg/ml):** | 50000.00 | 5000.00 | 1666.70 | 555.60 | 185.20 | 61.70 |
| **M (µg/ml):** | 900.00 | 300.00 | 100.00 | 33.30 | 11.10 | 3.70 |
| **Omega3 (µg/ml):** | 288.00 | 96.00 | 32.00 | 10.70 | 3.60 | 1.19 |
| **PEA (µg/ml):** | 26.90 | 9.00 | 3.00 | 1.00 | 0.30 | 0.11 |

**Table 2. Concentrations of solutions in combinations**

| **Combinatio n** | **Comp ounds** | **C1** | | **C2** | | **C3** | | **C4** | | **C5** | | **C6** |
|---|---|---|---|---|---|---|---|---|---|---|---|---|
| **Comb1** | E (µg/ml) | 25000.00 | | 2500.00 | | 833.35 | | 277.80 | | 92.60 | | 30.85 |
| M (µg/ml) | 450.00 | | 150.00 | | 50.00 | | 16.65 | | 5.55 | | 1.85 | |
| **Comb2** | E (µg/ml) | 16666.67 | | 1666.67 | | 555.57 | | 185.20 | | 61.73 | | 20.57 |
| M (µg/ml) | 300.00 | | 100.00 | | 33.33 | | 11.10 | | 3.70 | | 1.23 | |
| PEA (µg/ml) | 8.97 | | 3.00 | | 1.00 | | 0.33 | | 0.10 | | 0.04 | |
| **Comb3** | CBD (µg/ml) | 7.65 | | 2.55 | | 0.85 | | 0.28 | | 0.1 | | 0.03 |
| E (µg/ml) | 12500.00 | | 1250.00 | | 416.68 | | 138.90 | | 46.30 | | 15.43 | |
| M (µg/ml) | 225.00 | | 75.00 | | 25.00 | | 8.33 | | 2.78 | | 0.93 | |
| PEA(µg/ml) | 6.73 | | 2.25 | | 0.75 | | 0.25 | | 0.08 | | 0.03 | |
| **Comb4** | E (µg/ml) | 12500.00 | | 1250.00 | | 416.68 | | 138.90 | | 46.30 | | 15.43 |
| M (µg/ml) | 225.00 | | 75.00 | | 25.00 | | 8.33 | | 2.78 | | 0.93 | |
| Omega3 (µg/ml) | 72.00 | | 24.00 | | 8.00 | | 2.68 | | 0.90 | | 0.30 | |
| PEA(µg/ml) | 6.73 | | 2.25 | | 0.75 | | 0.25 | | 0.08 | | 0.03 | |
| **Comb5** | CBD (µg/ml) | 6.12 | | 2.04 | | 0.68 | | 0.22 | | 0.08 | | 0.03 |
| E (µg/ml) | 10000.00 | | 1000.00 | | 333.34 | | 111.12 | | 37.04 | | 12.34 | |
| M (µg/ml) | 180.00 | | 60.00 | | 20.00 | | 6.66 | | 2.22 | | 0.74 | |
| Omega3 (µg/ml) | 57.60 | | 19.20 | | 6.40 | | 2.14 | | 0.72 | | 0.24 | |
| PEA (µg/ml) | 5.38 | | 1.80 | | 0.60 | | 0.20 | | 0.06 | | 0.02 | |

### Cell culture

### Characteristics and cell culture of the human microglia cell line HMC3

The human HMC3 cell line (ATCC, USA) was established through SV40-dependent immortalization of a human fetal brain-derived primary microglia culture. Resting HMC3 cells were shown to be strongly positive for the microglia/macrophage marker IBA1, positive for the endotoxin receptor CD1 but negative for the astrocyte marker GFAP. Markers of activated microglia - namely MHCII - CD68 and CD11b were negative in resting HMC3 cells, but upregulated after activation by IFN-gamma (10 ng/ml, 24 h) (ATCC^{®}CRL-3304).

HMC3 cells were cultured in EMEM medium (Roth) supplemented with 10% fetal bovine serum (PAA) and antibiotics (50 U ml-1 penicillin / 50 µg ml-1 streptomycin) in 75cm2 culture flasks (Incubator 37°, 5% CO2, 95% humidity). Media was changed every two to three days. For sub-cultivation cells were treated with 0.05% trypsin,0.02% EDTA for 5 to 10 minutes. The split ratio was 1 to 3.

### RT-PCR

HMC3 cells were seeded in 6-well plates (500.000 cells/well) and treated as described under "Viability Assay" (see Report 1) for 8 hrs and 24 hrs. RNA was isolated, the RT-PCR was performed. The primers are described in **Table 3.** Fold changes were calculated as described earlier (Abdel-Aziz et al. 2015) using the Quantstudio 7.

**Table 3. List of Genes and TAQ-Man Primers**

| **Gene Name** | **Gene Symbol** | **Gene ID** | **TAQ-Man Primer ID** |
|---|---|---|---|
| Cannabinoid receptor 1 | CNR1 | 1268 | Hs01038522_s1 |
| Cannabinoid receptor 2 | CNR2 | 1269 | Hs05019229_s1 |
| Transient receptor potential cation channel subfamily V member 1 | TRPV1 | 7442 | Hs00218912_m 1 |
| Interleukin 6 | IL-6 | 3569 | Hs00985639_m 1 |
| Diacylglycerol Lipase alpha | DAGLA | 747 | Hs00391374_m 1 |
| N-acylphosphatidyl ethanolamine phospholipase D | NAPEPLD | 222236 | Hs00419593_m 1 |
| Glycerophospho diesterphosphod iesterase1 | GDE1 | 51573 | Hs00945232_m 1 |
| Monoglyceride Lipase | MGLL | 11343 | Hs00996004_m 1 |
| N-acylehaolamami ne acid amidase | NAAA | 27163 | Hs00384501_m 1 |
| Fatty acid amid Hydrolase | FAAH | 2166 | Hs01038664_m 1 |
| Eukaryotic 18S rRNA | RNA18S5 | - | Hs03928990_g1 |
| Endogenous Control (FAM /MGB) | | | |

### ELISAs

**ELISA** - **IL6:** Cells (50x103 cells/well) were seeded into 96-well plates and allowed to attach for 24 h. After 24 h cells were washed twice with PBS and serum-free medium was added for 24 h for the synchronization of the cells' stage. Then the medium was replaced with fresh serum-free media and treated for 24 h (total volume - 200 µl). The IL6 release was determined by the Human IL6 DuoSet ELISA (R&D Systems) according to the manufacturer's instructions.

**ELISA** - **FAAH, AEA, 2-AG:** Cells (300x103 cells/well) were seeded into 24-well plates and allowed to attach for 24 h. After 24 h cells were washed twice with PBS and serum-free medium was added for 24 h for the synchronization of the cells' stage. Then the medium was replaced with fresh serum-free media and treated for 24 h (total volume - 300 µl). All values are expressed as mean ±SEM of two independent experiments with two replicates each.

The amount of the **Human Fatty-Acid Amide Hydrolase (FAAH)** was determined using the Human Fatty-Acid Amide Hydrolase 1 (FAAH) ELISA Kit from Abbexa. This kit is based on a sandwich ELISA method and has a sensitivity of 47 pg/ml. Samples (100µl each) and standards (100µl each) were incubated for 1 h and 30 min at 37°C, followed by the direct addition of biotin-conjugated reagent (100 µl each) without washing in between and incubated for 1 h at 37°C. Then, HRP-conjugated reagent was added, followed by another incubation for 30 min at 37°C. Unbound conjugates were washed away using a wash buffer. The HRP enzymatic reaction was measured using TMB substrate. Wells containing sufficient FAAH produce a blue product, which turns yellow upon adding the acidic stop solution. The intensity of the yellow colour indicates the amount of FAAH bound to the plate. The optical density was measured spectrophotometrically at 450 nm.

FAAH could not be detected in our initial experiment (300.000 cells/ freeze thaw cycle according to the manufacturer's recommendations). The optical density of the samples was less than the ones of the standard curve (**Table 4**). Thus experiments with a higher number of cells (500.000 cells/well in 12-well plates) as well as with a different way of preparation of samples (sonification instead of a freeze/thaw cycle) were undertaken. Results are shown in **Table 5.**

**Table 4. FAAH assay with 300.000 cells/well. Cells were lysed by 3 freezing-thawing cycles**

| **FAAH, pg/ml** | | **Standard curve** | | **Samples** | | **Extraxts/ Drugs treatment** | |
|---|---|---|---|---|---|---|---|
| **A** | 5000 | | 3.0212 | | 0.1158 | | CBD-C4 |
| **B** | 2500 | | 2.0475 | | 0.119 | | CBD-C5 |
| **C** | 1250 | | 1.2272 | | 0.1119 | | Comb3-C4 |
| **D** | 625 | | 0.7073 | | 0.1369 | | Comb3-C5 |
| **E** | 312.5 | | 0.4283 | | 0.1106 | | M-C4 |
| **F** | 156.25 | | 0.2771 | | 0.1045 | | E-C4 |
| **G** | 78.125 | | 0.1519 | | 0.1201 | | E-C5 |
| **H** | Blank | | 0.117 | | 0.1186 | | Control |

**Table 5. FAAH assay with 500.000 cells/well. Cells were lysed by sonication. Additionally, the media of the control (H2) was tested for the presence of FAAH**

| **FAAH, pg/ml** | | **Standard curve** | | **Samples** | | **Extracs/dr ug treatment** | |
|---|---|---|---|---|---|---|---|
| **A** | 5000 | | 3.6112 | | 0.1084 | | Omega3 -C2 |
| **B** | 2500 | | 2.6459 | | 0.1183 | | Omega3 -C4 |
| **C** | 1250 | | 1.1646 | | 0.1153 | | Comb4-C2 |
| **D** | 625 | | 0.8906 | | 0.1297 | | Comb4-C4 |
| **E** | 312.5 | | 0.4118 | | 0.1906 | | Comb2-C2 |
| **F** | 156.25 | | 0.2915 | | 0.1181 | | Comb2-C4 |
| **G** | 78.125 | | 0.1782 | | 0.1085 | | Control |
| **H** | Blank | 0.0464 | | 2.4369 | | Control (Media) | |

| | | | | | | | |
|---|---|---|---|---|---|---|---|
| Like in the experiments with 300.000 cells/well, the cellular fraction did show very low values. Interestingly the media of the control showed a high presence of FAAH. | | | | | | | |

### Anandamide Assay

The release of the endocannabinoid anandamide (AEA) into the medium was determined by using the Human Anandamide (AEA) ELISA Kit from Abbexa. This kit is based on a competitive ELISA method. It has a sensitivity of <0.89 ng/ml. Standards (50 µl each), test samples (50 µl each), and biotin-conjugated reagent (50 µl each) were incubated for 1 h at 37°C, followed by washing. A competitive inhibition reaction occurs between the biotin-labelled AEA and the unlabeled AEA on the pre-coated antibody. Then HRP-conjugated reagent was added, followed by a second incubation for 30 min at 37°C. Unbound conjugates were washed away using the wash buffer. The HRP enzymatic reaction was measured using TMB as substrate. Wells with sufficient AEA produce a blue product, turning yellow after adding the acidic stop solution. The intensity of the yellow colour indicates the inversely proportional amount of bound AEA. The concentration of AEA was calculated by measuring the OD at 450 nm using a microplate reader.

### 2-Arachidonoylglycerol Assay

The release of 2-AG into the medium was determined using the Human 2-Arachidonoylglycerol (2-AG) ELISA Kit from MyBioSource. This kit is based on a sandwich ELISA method, and it has a sensitivity of

1.41 ng/ml. Standards (75µl each), test samples (75µl each), and biotin-conjugated reagent (25µl each) are incubated for 1 h at 37°C and followed by washing. Then, HRP-conjugated reagent is added, followed by another incubation for 30 min at 37°C. Unbound conjugates are washed away using a wash buffer. The HRP enzymatic reaction is measured using TMB substrate. Wells with sufficient AEA produce a blue product, turning yellow after adding an acidic stop solution. The intensity of the yellow color indicates the inversely proportional amount of bound AEA. The optical density was measured spectrophotometrically at 450 nm with correction at 540 nm.

### Synergy Calculations

Synergy was calculated according to the Chou-Talalay method as described earlier (Chou TC, 2010; Ulrich-Merzenich et al., 2019).

### Results

### IL-6 Release

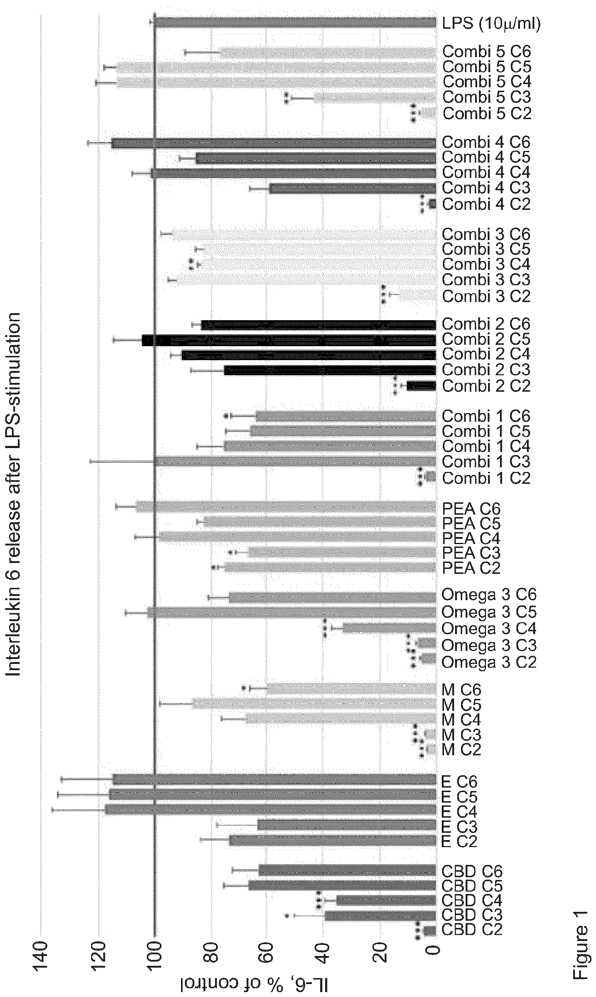

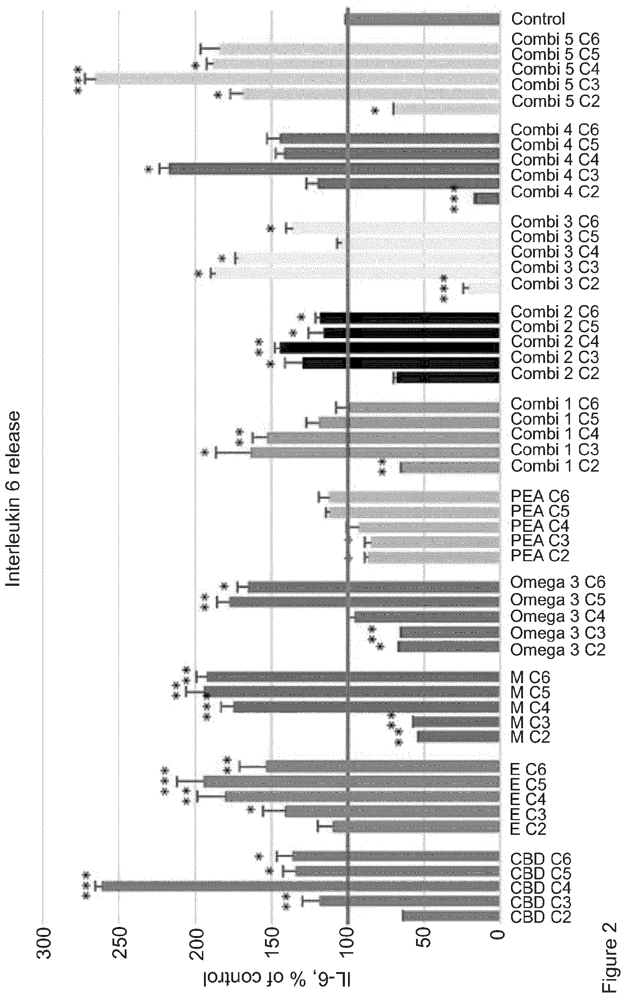

Supernatants of HMC3 cells treated with the different plant extracts/drugs were screened for their IL- 6 release under challenged and non-challenged conditions. Results are shown in **Figures 1** and **2****.**

In addition to investigating the inflammatory potential of plant extracts/drugs, experiments aimed also on the identification of one effective concentration for the gene expression analyses. The effective concentration was determined based on an IL-6 screening of the supernatants and C4 was used for gene expression analyses.

Figure 1 illustrates the release of IL-6 after treatment of the cells with plants' extracts/drugs and their combinations in the presence of LPS for 24 hours. Asterisks represent p-value: *** - p<0.001; ** - p<0.01; * - p<0.05.

The LPS-stimulation led to a mean release of 440.58 ± 22.09 pg/ml (Fig. 1). None of the single extracts nor the combinations led to an increase of the IL-6 release in the presence of LPS.

But the high concentrations (C2, C3) of the single extracts did significantly reduce the IL-6 release with the exception of the Echinaceae extract. However, the Echinaceae extract (C4-C6) did not significantly increase the IL-6 releases. Also, all combinations reduced in the highest concentration the IL-6 release significantly by about 90 %.

Figure 2 illustrates the release of IL-6 after treatment of the cells with plants' extracts/drugs and their combinations for 24 hours. Asterisks represent p-value: *** - p<0.001; ** - p<0.01; * - p<0.05.

The single extracts except PEA as well as the combinations did increase the IL-6 release in different concentrations. However, the mean release of IL-6 of the control was very low with 31.54 ± 1.63 pg/ ml. In comparison the LPS stimulation increased the IL-6 release to 440.58 ± 22.09 pg/ml. The induction of IL-6 by the single extracts led to a maximum release of 93 pg/ml.

### RT-PCR

The gene expression was measured in one concentration (C4) for each of the 5 plant extracts/drugs. However, since the experiments determining IL6 and the RT-PCR were performed in parallel / simultaneously not only 2 combinations, but all 5 combinations as well as all single drugs/extracts and the reference drug (+ control) were investigated. Since we could not detect an upregulation of CNR1 by CBD after 24 hrs in our initial experiments, it was agreed (after a joint meeting) that also the time point of 8 hrs should be investigated. In addition the number of PCR-cycles were increased from 40 to 45, thereby CNR1 and CNR2 could be detected upon CBD stimulation.

In additional pre-experiments to determine the experimental conditions we observed that CBD upregulated CNR1 and CNR2 after 1 h and 8 hrs concentration-dependently. At the time point 12 hrs this upregulation was already reduced. At time point 24 hrs no difference between CBD treatment and control samples could be detected.

The combinations 1, 2, 3 and 5 all increased the gene expression of CNR1 and CNR2 after 12 hrs to a variable extent. The gene expression of CNR2 was increased under the influence of combination 4 at 24 hrs, but not significantly. However, TRPV1 was significantly increased after 8hrs. Overall TRPV1 was well detectable at 1 h, 12 and 24hrs and was influenced by the single compounds and combinations differentially.

DAGLA, GDE1, MGLL, NAAA, NAPEPLD could all be detected at 8h, 12hr and 24 hrs under stimulation with CBD. For the experiments with all compounds and combinations the time points 8 hrs and 24 hrs were agreed upon as the most suitable.

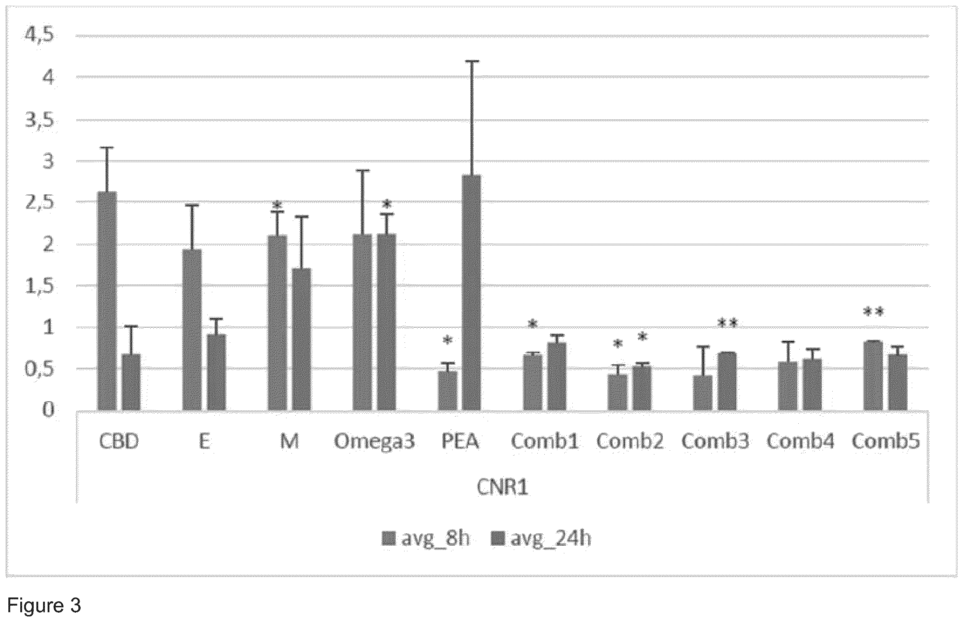

The Figures 3 to 11 show the results of the gene expression after 8 hrs and 24 hrs for all single extracts and all combinations treated with the concentration C4.

Figure 3 illustrates the expression of CNR1. Data are presented as 2^(-ddCt). Asterisks represent p-value: ** - p<0.01; * - p<0.05.

The concentration C4 was chosen for these experiments. All experiments were performed at least twice with 3 replicates.

CBD (1.1 µg/ml), the Echinacea-Extract (555.6 µg/ml), the Magnolia extract (33.3 µg/ml) and Omega- 3 fatty acids (10.7 µg/ml) increased the gene expression of the CNR1 receptor after 8hrs 2 to 2.5 fold. PEA (1 µg/ml) did increase the gene expression of the CNR1 receptor after 8 hrs significantly. The combinations had a tendency to decrease the CNR1 gene expression at both time points (Fig 3).

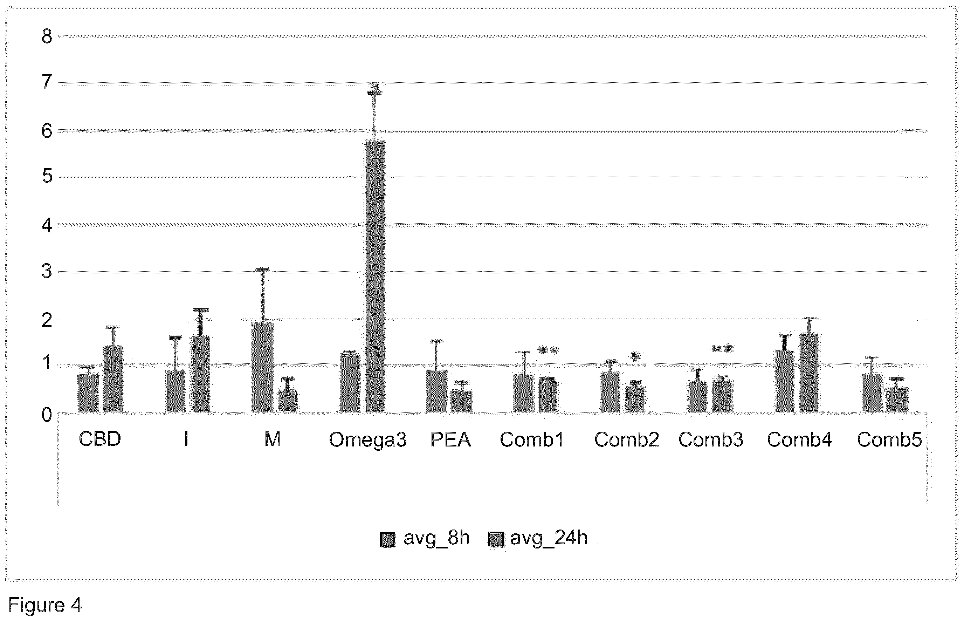

Figure 4 illustrates the Expression of CNR2. Data are represented as 2^(-ddCt). Asterisks represent p-value: ** - p<0.01; * - p<0.05.

The concentration C4 was chosen for these experiments. All experiments were performed at least twice with 3 replicates.

CBD (1.1 µg/ml), the Echinacea-Extract (555.6 µg/ml) and Omega-3 fatty acids (10.7 µg/ml) increased the gene expression of the CNR2 receptor after 24hrs 1.5 to 5 fold. The Magnolia-Extract (33.3 µg/ml) increases the gene expression of the CNR2 after 24 hrs. PEA (1.0 µg/ml) did not increase the gene expression of the CNR2 receptor. The combination 4 increases the CNR2 gene expression after 8 hrs and 24 hrs. None of the combinations except combination 4 increased the CNR2 expression under these experimental conditions (Fig 4).

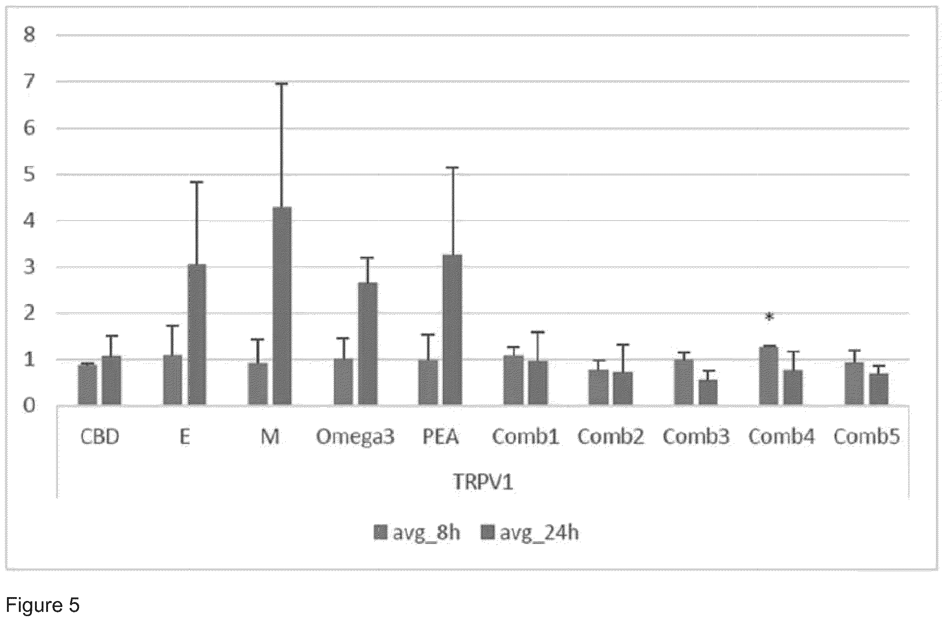

Figure 5 illustrates the expression of TRPV1. Data are represented as 2^(-ddCt). Asterisks represent p-value: ** - p<0.01; * - p<0.05.

The Echinacea extract (555.6 µg/ml), the Magnolia extract (33.3 µg/ml), Omega-3 fatty acids (10.7 µg/ml) and PEA (1.0 µg/ml) increased the gene expression of the TRVP1 receptor after 24hrs approximately 3- to 4-fold. Combination 4 slightly increased the gene expression of the TRPV1 receptor after 8 hrs. The other combinations did not increase the gene expression of the TRPV1 receptor under these experimental conditions (Fig 5).

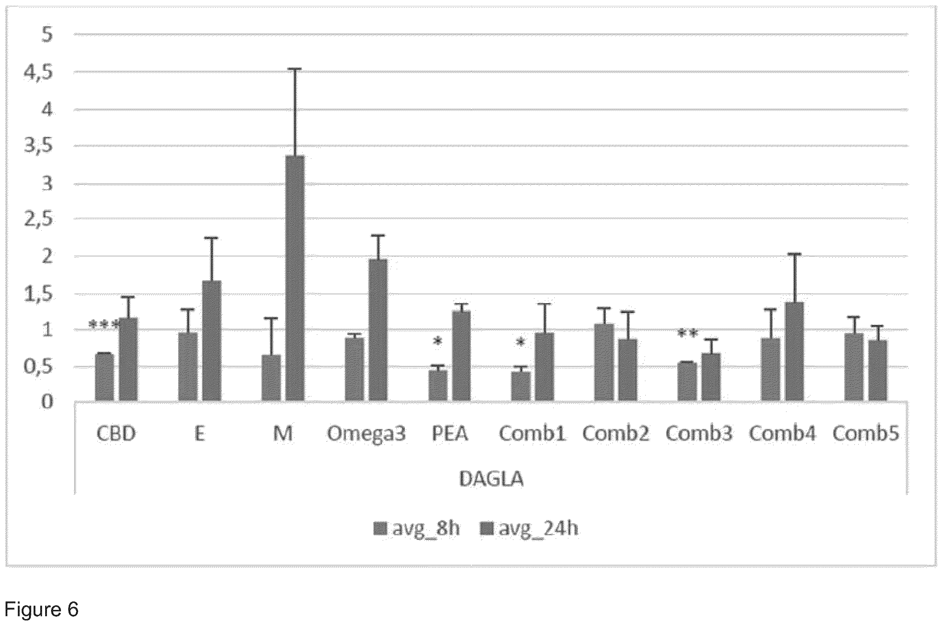

Figure 6 illustrates the expression of DAGLA. Data are represented as 2^(-ddCt). Asterisks represent p-value: ** - p<0.01; * - p<0.05.

The gene expression of the anabolic enzyme diacyl glycerol lipase (DAGL) was increased by all single extracts after 24 hrs incubation, but also slightly by the combinations 2,3, 4 and 5 after 24 hrs (Concentration C4) (Fig 6). The encoded gene is involved in the biosynthesis of endocannabinoids.

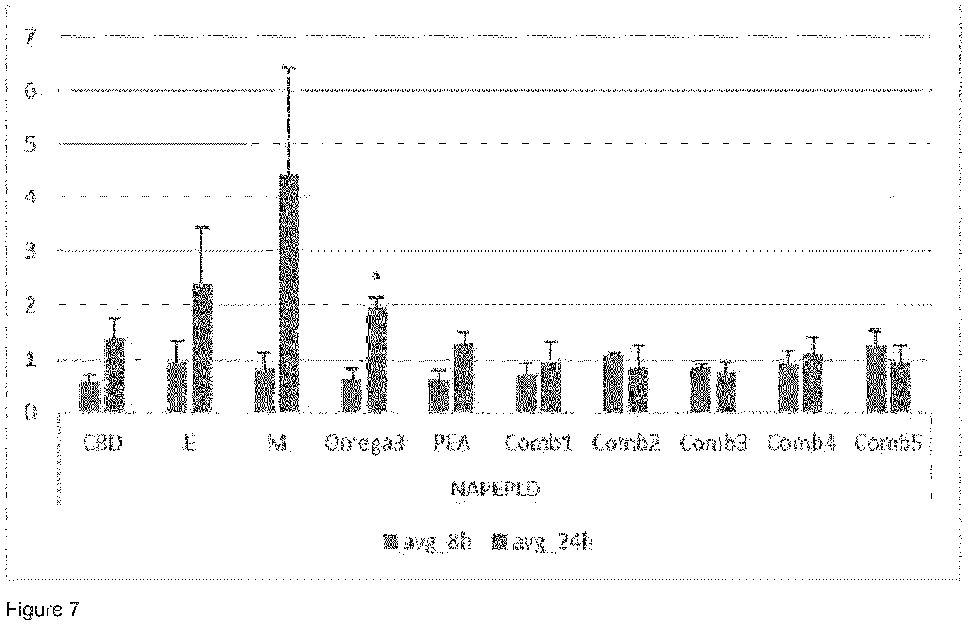

Figure 7 illustrates the expression of NAPEPLD. Data are presented as 2^(-ddCt). Asterisks represent p-value: ** - p<0.01; * - p<0.05.

The gene expression of N-acyl phosphatidylethanolamine phospholipase D (NAPE-PLD) was increased by the single extracts/drugs (CBD, E, M and Omega fatty acids), but not affected by the combinations (Fig 7). NAPEPLD generates the formation of anandamide by the cleavage of its membrane bound precursor N-arachidonoyl phosphatidylethanolamine. Its presence has been demonstrated in the brain (highest RPKM), but also in the stomach and in the small intestine

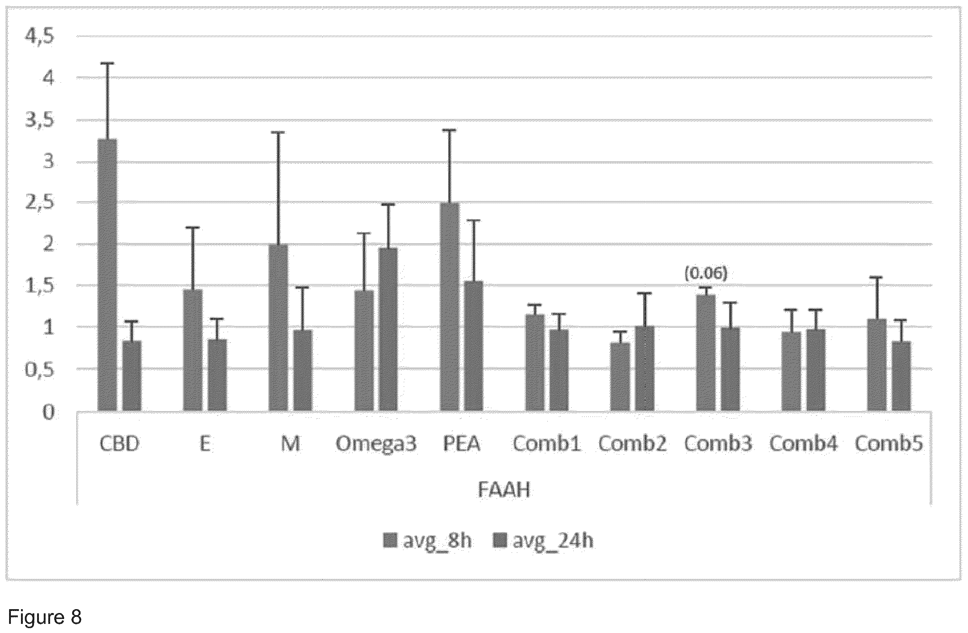

Figure 8 illustrates the expression of FAAH. Data are presented as 2^(-ddCt). (0.06). Asterisks represents p-value.

The gene expression of the fatty acid amide hydrolase (FAAH) is increased by CBD, E, M, Omega fatty acid and PEA after 8hrs, which seems to be a transient effect in case of CBD, E and M. Combi 4 did not increase the gene expression of FAAH, neither after 8 nor after 24 hrs but also the combination 2 did not increase the gene expression of FAAH - neither at 8 hrs nor at 24 hrs (Fig 8) (see also results of FAAH ELISA) (Fig 16 and 17).

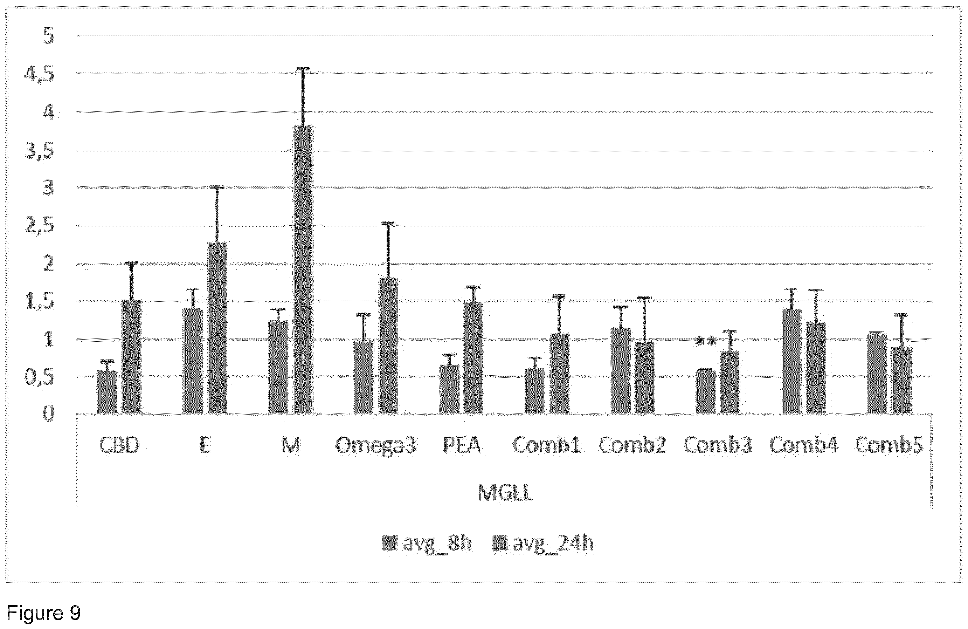

Figure 9 illustrates the expression of MGL. Data are represented as 2^(-ddCt). Asterisks represent p-value<0.01.

The gene expression of the monoacyl glycerol lipase (MGLL) was increased by all single substances / extracts and slightly increased by combination 2 after 8hrs. (Fig 9). MGLL is considered to play a central role in pain and nociperception through the hydrolysis of endocannabinoid 2-AG (2- arachidonoylglycerol). The gene is expressed in several tissues. Of special relevance to this project may be the gene expression in the brain, in the esophagus (much higher compared to brain), in the stomach, in fat tissue and in the small intestines. Higher expression of MAGL in gray and white matter was associated with a cannabis dependency, a potentially restricted cannabinoid signaling and a higher vulnerability to chances in cortical thickness while consuming cannabis.

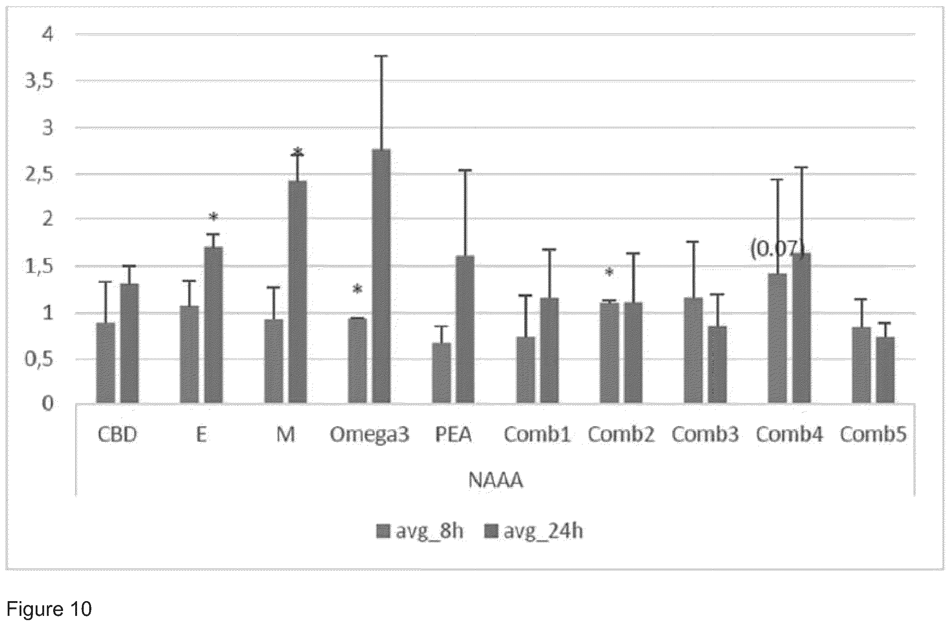

Figure 10 illustrates the expression of NAAA. Data are presented as 2^(-ddCt). Asterisks represent p-value <0.05. For Comb4 p-value = 0.07.

The gene expression of the N-acylethanolamine acid amidase (NAAA) is increased by the single extracts and CBD after 24 hrs as well as by combination 4 after 8 and 24 hrs but not by the combination 1, 2, 3 and 5 (Fig 10). NAAA is less expressed in the human brain but in the colon (highly), in the esophagus, the stomach and in the small intestine (high) as well as in other tissues (source: NCBI gene data base). Acid ceramidases are proposed to play a role in neuronal apoptosis.

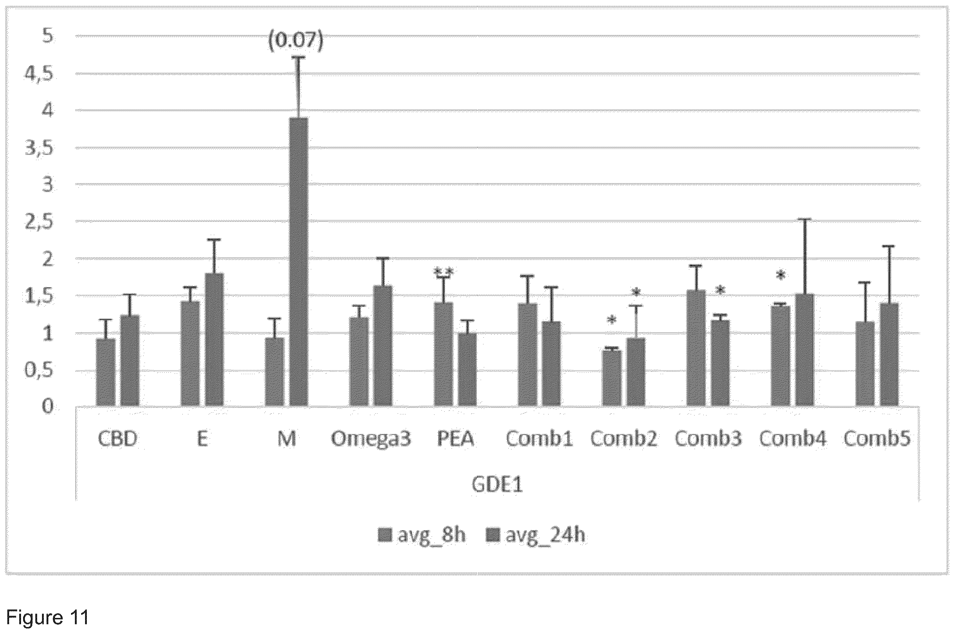

Figure 11 illustrates the expression of GDE. Data are represented as 2^(-ddCt). Asterisks represent p-value: ** - p<0.01; * - p<0.05. For M (24 h) p-value = 0.07.

The gene expression of the glycerophosphodiester phosphodiesterase 1 (GDE1) was increased by CBD, E, M and Omega fatty acids after 24 hrs. There was a slight increase by Combination 4 after 24 hrs and by the combination 3 after 8 hrs. Combinations 2 rather decreased the gene expression of GDE1 (Fig 11). GDE1 is proposed to be involved into metabolic processes of endocannabinoids (Fezza F. et al., 2014).

The following Tables 6 to 10 provide a summary of the gene expressions of the different combinations in comparison to the single extracts/drugs at 24 hrs

**Table 6. Combination 1 (24 hrs)**

| **Gene** | **M** | **E** | **Combi 1 (M+E)** |
|---|---|---|---|
| CNR1 | ↑ | - | - |
| CNR2 | ↓ | ↑ | ↓ |
| TRPV1 | ↑ | ↑ | - |
| DAGL | ↑ | ↑ | - |
| NAPE-PLD | - | - | - |
| FAAH | - | - | - |
| MGLL | ↑ | ↑ | - |
| NAAA | ↑ | ↑ | - |
| GDE1 | ↑ | ↑ | - |

**Table 7. Combination 2 (24 hrs)**

| **Gene** | **M** | **E** | **PEA** | **Combi 2 (M+E+PEA)** |
|---|---|---|---|---|
| CNR1 | ↑ | - | ↑ | ↓ |
| CNR2 | ↓ | ↑ | ↓ | ↓ |
| TRPV1 | ↑ | ↑ | ↑ | - |
| DAGL | ↑ | ↑ | ↑ | - |
| NAPE-PLD | - | - | ↑ | - |
| FAAH | - | - | ↑ | - |
| MGLL | ↑ | ↑ | ↑ | - |
| NAAA | ↑ | ↑ | ↑ | - |
| GDE1 | ↑ | ↑ | - | ↑ |

**Table 8. Combination 3 (24 hrs)**

| **Gene** | **M** | **E** | **PEA** | **CBD** | **Combi 3 (M+E+PEA+CBD)** |
|---|---|---|---|---|---|
| CNR1 | ↑ | - | ↑ | ↓ | ↓ |
| CNR2 | ↓ | ↑ | ↓ | - | ↓ |
| TRPV1 | ↑ | ↑ | ↑ | - | -- |
| DAGL | ↑ | ↑ | ↑ | ↑ | - |
| NAPE-PLD | - | - | ↑ | ↑ | - |
| FAAH | - | - | ↑ | - | -- |
| MGLL | ↑ | ↑ | ↑ | - | -- |
| NAAA | ↑ | ↑ | ↑ | - | - |
| GDE1 | ↑ | ↑ | - | - | ↑ |

**Table 9. Combination 4 (24 hrs)**

| **Gene** | **M** | **E** | **PEA** | **-3FS** | **Combi 4 (M+E+ PEA+ 3-FS)** |
|---|---|---|---|---|---|
| CNR1 | ↑ | - | ↑ | ↑ | - |
| CNR2 | + | ↑ | ↓ | ↑ | ↑ns |
| TRPV1 | ↑ | ↑ | ↑ | ↑ns | - |
| DAGL | ↑ | ↑ | ↑ | ↑ns | ↑ns |
| NAPE-PLD | - | - | ↑ | ↑ | - |
| FAAH | - | - | ↑ | ↑ns | -- |
| MGLL | ↑ | ↑ | ↑ | ↑ns | ↑ns |
| NAAA | ↑ | ↑ | ↑ | ↑ns | ↑ns |
| GDE1 | ↑ | ↑ | - | ↑ns | ↑ns |

**Table 10. Combination 5 (24 hrs)**

| **Gene** | **M** | **E** | **PE A** | **-3 FS** | **CBD** | **Combi 5 (M+E+ PEA+ 3-FS, CBD)** |
|---|---|---|---|---|---|---|
| CNR1 | ↑ | - | ↑ | ↑ | ↓ | - |
| CNR2 | ↓ | ↑ | ↓ | ↑ | - | ↓ns |
| TRPV1 | ↑ | ↑ | ↑ | ↑ns | - | ↓ns |
| DAGL | ↑ | ↑ | ↑ | ↑ns | ↑ | - |
| NAPE-PLD | - | - | ↑ | ↑ | ↑ | - |
| FAAH | - | - | ↑ | ↑ns | - | - |
| MGLL | ↑ | ↑ | ↑ | ↑ns | - | - |
| NAAA | ↑ | ↑ | ↑ | ↑ns | - | - |
| GDE1 | ↑ | ↑ | - | ↑ns | - | ↑ns |

The expression of genes relevant to the endocannabinoid system and stimulated by the single extracts/drug for 24 hrs in microglia cells did not show synergistic or additive effects in the combinations. In most cases effects seemed to cancel each other out in the combination. But increases in e.g., the anandamide or the 2-AG releases were measurable. Thus, the gene expression measured after 24 hrs may not in all cases be representative for the current events. This is further supported by the data after 8 hrs which show e.g., for CBD an increase in the gene expression of the CNR2. These experimental results could not be foreseen from literature.

Interestingly PEA upregulated most of the selected genes relevant for the endocannabioid system. PEA itself is an endocannabinoid like mediator with indeed extensively documented anti-inflammatory, analgesic, antimicrobial, immunomodulatory and neuroprotective effects

### The Endocannabinoid Anandamide

The endocannabinoid arachidonoyl ethanolamine (anandamide) is a lipid transmitter synthesized and released "on demand" by neurons in the brain (Liu et al. 2006). Anandamide can also be generated by macrophages after stimulation with LPS. Furthermore, anandamide can be generated by its membrane precursor N-arachidonoyl phosphatidylethanolamine (NAPE) through cleavage by a phospholipase D (NAPE-PLD).

Anandamide as an immune modulator in the central nervous system acts not only via the cannabinoid receptors (CB1, CB2) but also via other targets e.g. GPR18 and GPR55. Both are regarded now as endocannabinoid receptors.

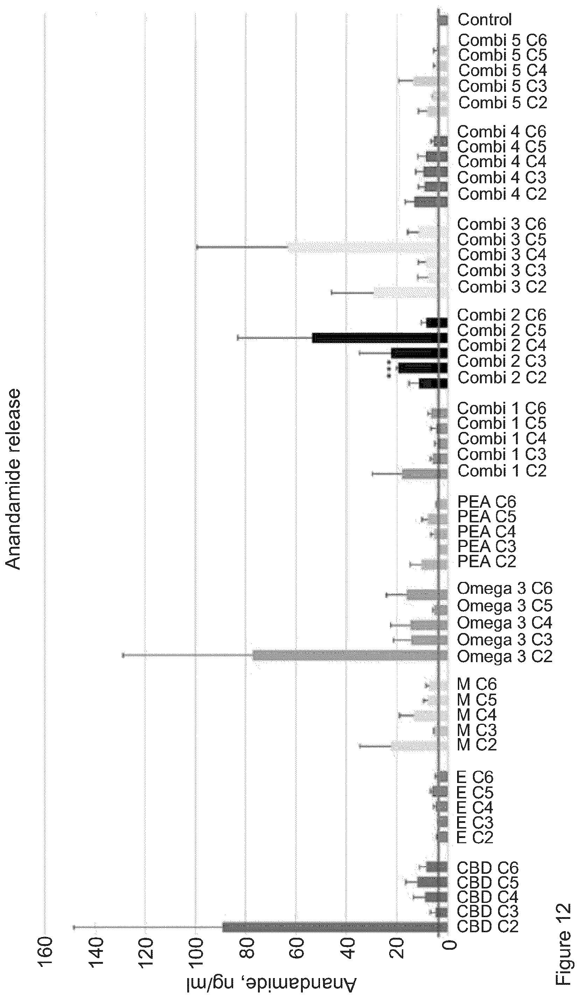

The anandamide release by the HMC3 cells is illustrated in four graphs (Fig. 12 to Fig. 15). Figure 12 provides an overview and illustrates the release of Anandamide after treatment of the cells with plant extracts/drugs and their combinations for 24 hours. Asterisks represent p-value: *** - p<0.001; ** - p<0.01; * - p<0.05.

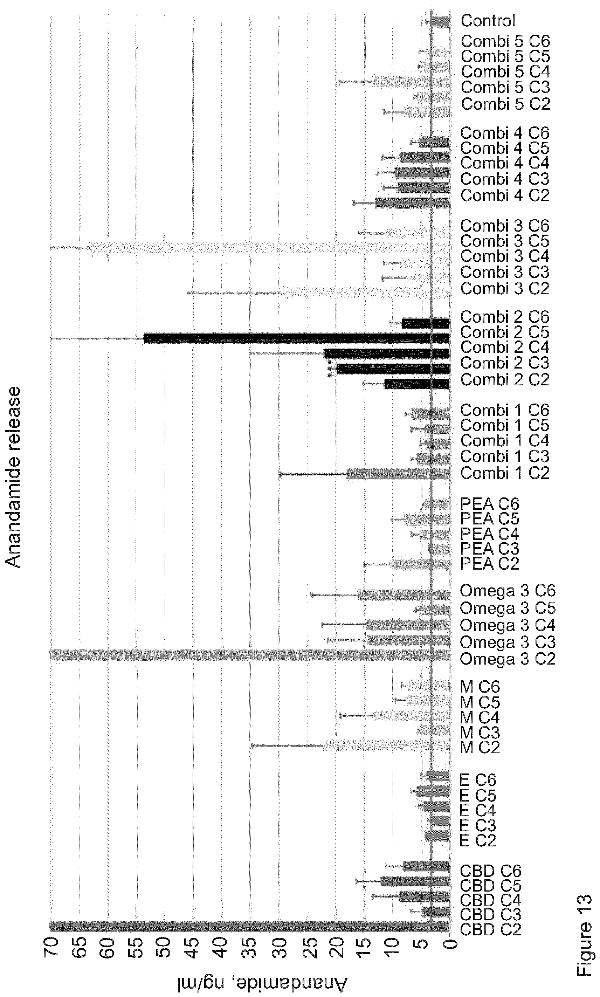

Figure 13 shows enlargements of the anandamide release up to 70 ng/ml to provide a better overview about the lower releases induced by the drugs/extracts and combinations. This is a magnified Bar Chart of the Anandamide release (Fig. 12) after treatment of the cells with plant extracts/drugs and their combinations for 24 hours. Asterisks represent p-value: *** - p<0.001; ** - p<0.01; * - p<0.05.

The highest concentrations of CBD, of the Magnolia extract, of the omega3 fatty acids and of PEA increased the anandamide release substantially. This was not the case for the Echinaceae extract. Here none of the selected concentrations did induce an anandamide release. The Magnolia extract and the omega 3 FS did increase the anandamide release in various concentrations.

Only the highest concentration of combination 1 did increase the anandamide release. Combination 2 and combination 3 increased the Anandamide release substantially in more than one concentration. Combination 4 and 5 also increased the Anandamide release in some concentrations, however to a lower extent.

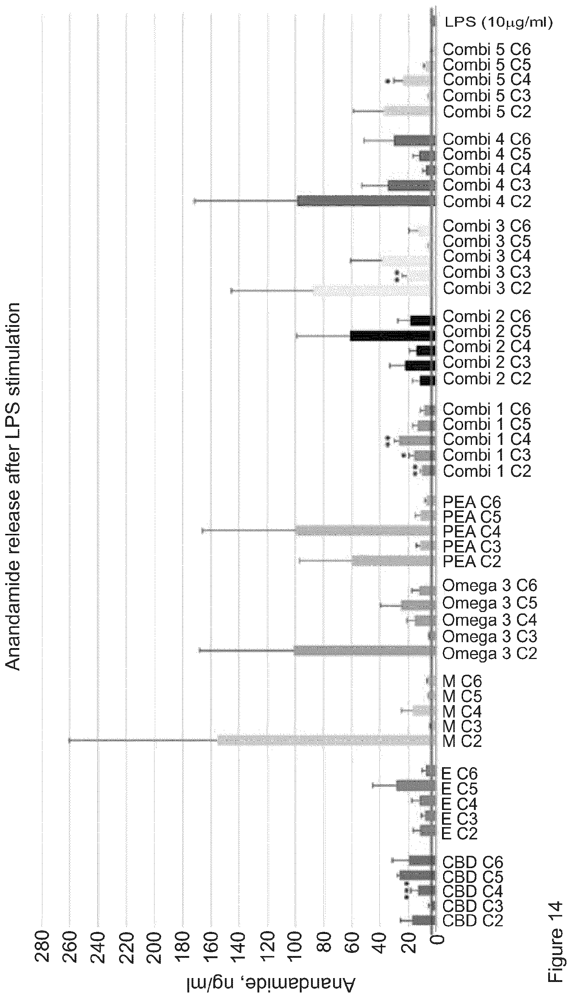

Figure 14 illustrates the expression of Anandamide after treatment of the cells with plant extracts/drugs and their combinations in the presence of LPS for 24 hours. Asterisks represent p-value: *** - p<0.001; ** - p<0.01; * - p<0.05.

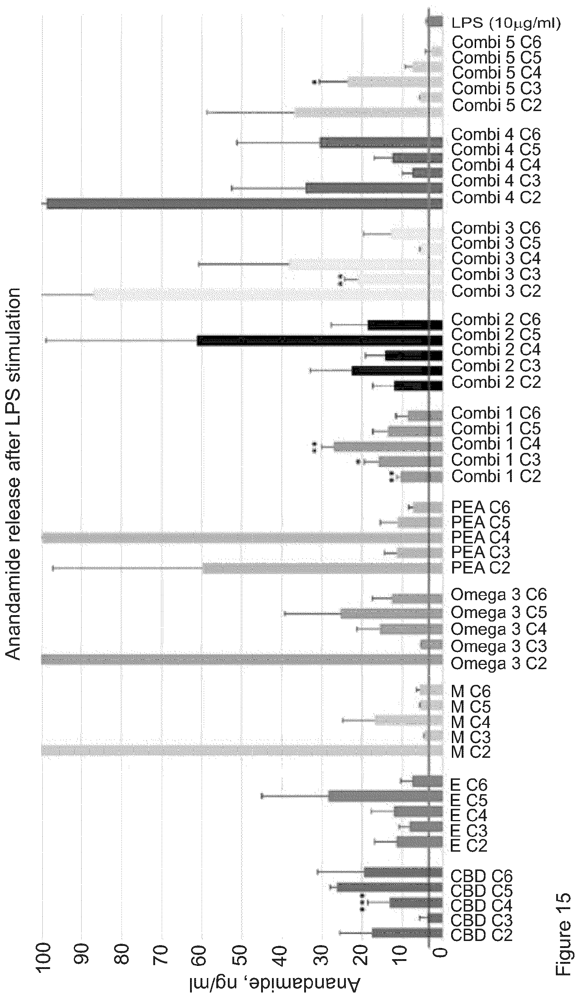

Figure 15 is a magnified Bar Chart of Anandamide release (Fig. 14) after treatment of the cells with plant extracts/drugs and their combinations with addition of LPS stimulation for 24 hours. Asterisks represent p-value: *** - p<0.001; ** - p<0.01; * - p<0.05.

The stimulation with LPS increased the anandamide release up to 6.6 ng/ml compared to the highest control with a release of 3.66ng/ml. Like in the unchallenged experiment, the highest concentration C2 induced a high release of anandamide in the cases of the single extracts of Mangnolia, the omega3 FS and PEA. For the combination this was only the case for combination 3, the combination 4 and the combination 5. Under challenged conditions the combinations 2, 3 and 4 showed the highest anandamide releases. But like under unchallenged conditions high variations were present and a higher number of experiments is required to substantiate the results.

FAAH assay: A minimum of 5 concentrations for each plant/extract was assessed under challenged and non-challenged conditions. All experiments were performed in duplicates with two replicates each.

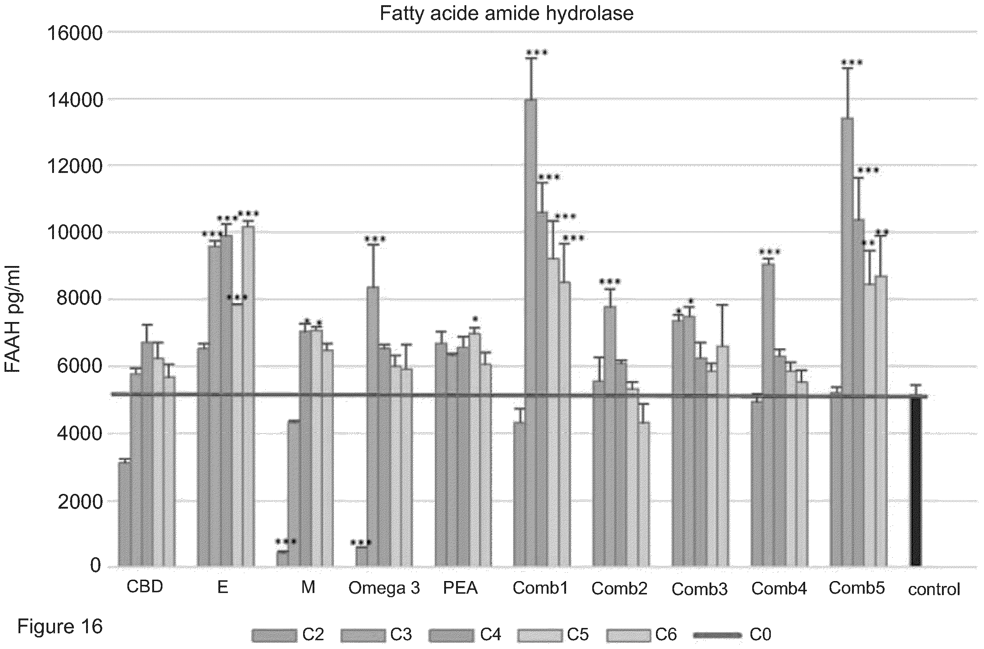

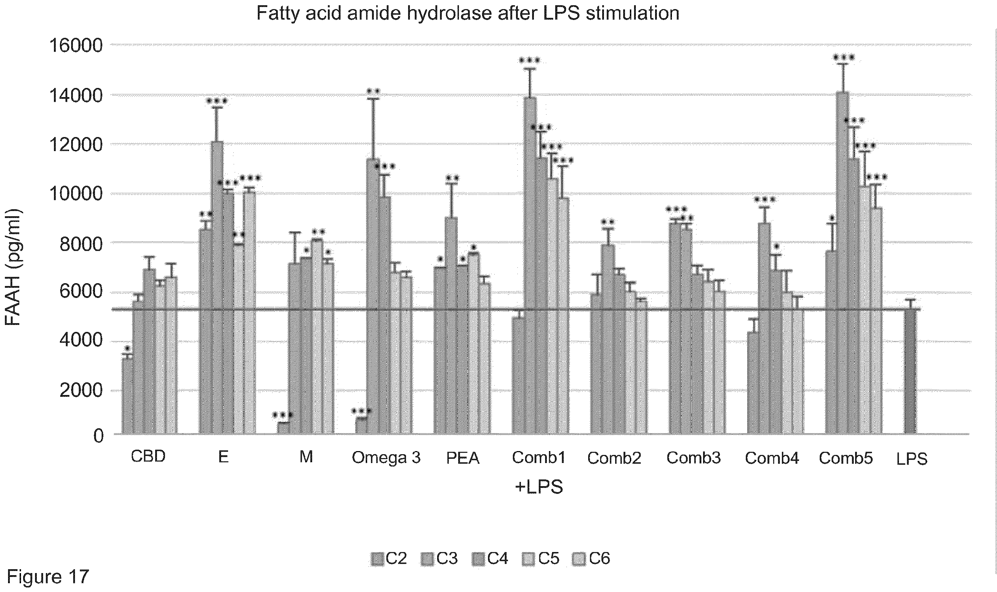

Figures. 16 and 17 show the amount of Fatty acid amide hydrolase induced by the different plant extracts/drugs under challenged conditions (LPS) and non-challenged conditions.

Figure 16 illustrates the expression of Fatty acid amide hydrolase after treatment of the cells with plant extracts/drugs and their combinations for 24 hours. Asterisks represent p-value: *** - p<0.001; ** - p<0.01; * - p<0.05.

It is striking that especially the highest concentration (C2) did inhibit the FAAH while simultaneously the anandamide release (see Fig. 12 to 15) is increased. This is the case for CBD, the Magnolia extract and Omega3 fatty acids and to a lesser extent for the combination 1 (C2) and the combination 2 (C5).

Figure 17 illustrates the expression of Fatty acid amide hydrolase after treatment of the cells with plant extracts/drugs and their combinations and with addition of LPS stimulation for 24 hours. Asterisks represent p-value: *** - p<0.001; ** - p<0.01; * - p<0.05.

LPS did only slightly increase the amount of FAAH. Therefore results are quite similar to the unchallenged condition. The highest concentrations of CBD, M and Omega3 did significantly reduce the presence of FAAH. Other concentration of the single extracts did increase FAAH to a variable extent. In case of the combinations, only C3 of combination 2 did additionally increase FAAH.

### Assessment of 2-arachidonylglycerol (2-AG)

2-AG activates as endocannabinoid the CB1 and CB2 receptors.

Figure 18 illustrates the expression of 2-Arachidonoylglycerol formation after treatment of the cells with plant extracts/drugs and their combinations for 24 hours. Asterisks represent p-value: * - p<0.05.

Figure 19 illustrates the expression of. 2-Arachidonoylglycerol formation after treatment of the cells with plant extracts/drugs and their combinations and with addition of LPS stimulation for 24 hours. Asterisks represent p-value: * - p<0.05.

CBD, especially the Mangnolia extract, PEA and the combinations 1, 4 and 5 increase in concentration 4 the release of 2-AG. The LPS stimulation increased the 2-AG slightly from 3 ± ng/ml to 3.5± ng/ml. Under these challenged conditions also Omega3 FS increased the 2-AG releases (besides CBD, M and PEA). The treatment of all combinations in at least one concentration led to a significant increase in the 2-AG release. However, compared to the anandamide releases, the effects are much smaller.

### Synergy Assessments

Data were analyzed for their synergistic/antagonistic or additive effect by the combination Index (CI) method. Tables 11 and 12 summarizes the Cls of the combinations for the different effect levels with respect to the different assays. Two principal calculations were performed: a) all measured values were considered (Table 11) b) only values meeting the requirements of a dose-response curve were considered (Table 12)

### Synergy assessments considering all measured values

**Table 11. Summary of the synergy assessments for the different assays (all data)**

| **Assay** | **Comb1** | **Comb2** | **Comb3** | **Comb4** | **Comb5** |
|---|---|---|---|---|---|
| | **(M,E)** | **(M,E,PEA)** | **(M,E,PEA,CBD)** | **(M,E,PEA, -3)** | **(M,E,PEA, - 3,CBD)** |
| **FAAH** | CI=0.03 (Fa=0.5) | Antagonism* | Antagonism* | Antagonism* | CI=3.91E-6 |
| | | | | | (Fa=0.5) |
| **FAAH + LPS** | CI=2.5E-26 (Fa=0.9) | Antagonism* | Antagonism* | Antagonism* | Antagonism* |
| | CI=3.1E-47 (Fa=0.95) | | | | |
| | CI=4.2E-62 (Fa=0.97) | | | | |

| **AEA** | Antagonism* | CI=8.48E-7 (Fa=0.5) | CI=8.17E-4 (Fa=0.5) | Antagonism* | Antagonism* |
|---|---|---|---|---|---|
| | | CI=2.5E-13 (Fa=0.75) | CI=1.06E-7 (Fa=0.75) | | |
| | | Cl=7.7E-20 (Fa=0.9) | CI=1.4E-11 (Fa=0.9) | | |
| | | CI=2.8E-24 (Fa=0.95) | CI=3.1E-14 (Fa=0.95) | | |
| | | CI=2E-25 (Fa=0.97) | CI=4.1E-16 (Fa=0.97) | | |
| **AEA+L PS** | Antagonism* | Antagonism* | Antagonism* | Antagonism* | Antagonism |
| **2-AG** | CI=0.11 (Fa=0.65) | Antagonism* | Antagonism* | Antagonism* | Antagonism |
| **2-AG+** | Antagonism* | Antagonism* | Antagonism* | Antagonism* | CI=0.11 |
| **LPS** | | | | | (Fa=0.5) |

| | | | | | |
|---|---|---|---|---|---|
| *CI-Values below Fa=0.5 (Fraction affected 50%) were not considered. | | | | | |

Especially the combinations 2 and 3 show synergistic effects with respect to the Anandamide release especially considering higher effect levels (50 to 97 %).

### Synergy assessments after removal of data that are not meeting the requirement of a dose- response relationship

**Table 12. Summary of the synergy assessments for the different assays (dose-response data)**

| **Assay** | **Comb1** | **Comb2** | **Comb3** | **Comb4** | **Comb5** |
|---|---|---|---|---|---|
| | **(M,E)** | **(M,E,PEA)** | **(M,E,PEA,CBD)** | **(M,E,PEA, -3)** | **(M,E,PEA, - 3,CBD)** |
| **FAAH** | CI=0.02 (Fa=0.5) | CI=0.04 (Fa=0.5) | CI=0.62 (Fa=0.5) | CI=0.48 (Fa=0.5) | CI=0.62 (Fa=0.5) |
| | CI=0.22 (Fa=0.75) | | | | |
| **FAAH+ LPS** | CI=0.26 (Fa=0.5) | Antagonism | CI=0.83 (Fa=0.5) | Antagonism | CI=0.55 (Fa=0.5) |
| | CI=0.22 (Fa=0.75) | | | | |
| **AEA** | Cl=1.2E-11 (Fa=0.5) | Antagonism | CI=2.52E-4 (Fa=0.5) | Antagonism | Antagonism |
| | | | CI=7.99E-6 (Fa=0.75) | | |
| | CI=9.5E-15 (Fa=0.75) | | | | |
| | | | CI=2.6E-7 (Fa=0.9) | | |
| | CI=7.6E-18 (Fa=0.9) | | CI=2.55E-8 (Fa=0.95) | | |
| | CI=5.9E-20 (Fa=0.95) | | CI=4.89E-9 (Fa=0.97) | | |
| | CI=1.9E-21 (Fa=0.97) | | | | |
| **AEA+L PS** | Antagonis m | CI=0.16 (Fa=0.5) | Antagonism | Antagonism | Antagonism |
| | | CI=0.003 (Fa=0.75) | | | |
| | | CI=6.45E-5 (Fa=0.9) | | | |
| | | CI=4.51E-6 (Fa=0.95) | | | |
| | | CI=6.82E-7 (Fa=0.97) | | | |
| **2-AG** | CI=0.05 (Fa=0.75) | Antagonism | Antagonism | Antagonism | Antagonism |
| | CI=0.001 (Fa=0.9) | | | | |
| | CI=1.06E-4 (Fa=0.95) | | | | |
| | CI=1.91E-5 (Fa=0.97) | | | | |
| **2-AG+LP S** | CI=0.46 (Fa=0.5) | CI=0.79 (Fa=0.75) | CI=0.02 (Fa=0.75) | CI=0.004 (Fa=0.75) | CI=0.5 (Fa=0.75) |
| | CI=0.26 (Fa=0.75) | CI=0.31 (Fa=0.9) | CI=6.08E-4 (Fa=0.9) | CI=6.36E-5 (Fa=0.9) | |
| | CI=0.18 (Fa=0.9) | CI=0.18 (Fa=0.95) | CI=6.11E-5 (Fa=0.95) | CI=4.52E-6 (Fa=0.95) | |
| | CI=0.14 (Fa=0.95) | CI=0.13 (Fa=0.97) | CI=1.2E-5 (Fa=0.97) | CI=7.11E-7 | |
| | CI=0.12 (Fa=0.97) | | | (Fa=0.97) | |

All combinations show synergistic effects with respect to the 2-AG release under challenged conditions. Particularly combination 2 shows synergistic effects also with respect to the release of anandamide under challenged conditions.

Overall, the chosen drugs/plant extracts and the five combinations thereof influence the endocannabinoid system. Table 13 provides an overview of the results for the different combinations in all performed assays with an evaluation of their effects on the endocannabinoid system.

**Table 13. Comparative overview of the results of all assays for all combinations**

| **Assay** | **Comb1** | **Comb2** | **Comb3** | **Comb4** | **Comb5** | CBD |
|---|---|---|---|---|---|---|
| | **(M,E)** | **(M,E,PEA)** | **(M,E,PEA, CBD)** | **(M,E,PE A, - 3)** | **(M,E,PE A, - 3,CBD)** | |
| **ELISA** | | | | | | |
| AEA | Increase ns | **Increase** | Increase ns | Increase ns | Increase ns | Increase |
| AEA+LPS | **Increase** | Increase | **Increase** | Increase ns | Increase ns | Increase ns |
| FAAH | Increase ++++ | Increase + | Increase ++ | Increase + | Increase ++++ | Increase ns |
| FAAH+LPS | Increase ++++ | Increase + | Increase ++ | Increase ++ | Increase ++++ | Increase ns |
| 2-AG | Increase ns | Increase ns | - | Increase ns | Increase ns | Increase ++ |
| 2-AG+LPS | Increase ns | Increase ns | Increase ns | Increase ns | Increase ns | Increase ns |

| **Synergy** | | | | | | |
|---|---|---|---|---|---|---|
| AEA | + | - | + | - | - | |
| AEA+LPS | - | + | - | - | - | |
| FAAH | + | + | + | + | + | |
| FAAH+LPS | - | + | - | - | - | |
| 2-AG | + | - | - | - | - | |
| 2-AG+LPS | + | + | + | + | + | |

| **Geneexpre ssion** | | | | | | |
|---|---|---|---|---|---|---|
| CNR1 | 8h downreg. | 8h downreg. | 8h downreg | 8h downreg. | 8h downr. | ns |
| CNR2 | 24h downreg | 24 h downreg. | 24h downreg | ns | ns | ns |
| TRPV1 | ns | Ns | ns | 8h upreg. | ns | ns |
| DAGL | 8h downreg. | Ns | 8h downreg | ns | ns | 8h downreg |
| NAPE-PLD | ns | Ns | ns | ns | ns | ns |
| FAAH | 8h slight upreg ns | Ns | 8h slight upreg ns | ns | ns | 8 h upreg. |
| MGLL | ns | Ns | 8h downreg | upreg ns | ns | ns |
| NAAA | ns | **8h upreg.** | ns | upreg. ns | downreg. ns | ns |
| GDE1 | ns | 8h downreg | upreg ns | 8h upreg | upreg ns | ns |

The interesting and somewhat stunning result was that the combinations increased (albeit not always significantly) the anandamide and 2-AG releases in microglia cells while also the FAAH levels increased. The anandamide release in the supernatant, especially in the extreme case of stimulations with C2, corresponded to the FAAH measurements: low anandamide corresponded with high FAAH and vice versa. An FAAH inhibiting activity was not observed for the combinations at the chosen experimental conditions. Combination 2 performed best, considering the release of anandamide and the measured FAAH. An increased release of anandamide was especially stimulated by combination 2.

## Claims

1. A composition comprising extract of *Echinaceae purpurea L,* extract of *Mangnolia officinalis* and Palmitoylethanolamide for use in a method of treatment,
wherein the method of treatment comprises administering said composition to a person in need thereof.

2. A composition according to claim 1, wherein the concentration of extract of *Echinaceae purpurea L* is in the range from 1 % by weight to 50 % by weight based on the total weight of the composition.

3. A composition according to claim 1 or 2, wherein the concentration of extract of *Mangnolia officinalis* is in the range from 1 % by weight to 50 % by weight based on the total weight of the composition.

4. A composition according one of the preceding claims, wherein the concentration of Palmitoylethanolamide is in the range from 1 % by weight to 50 % by weight based on the total weight of the composition.

5. A composition according one of the preceding claims, wherein the composition further comprises cannabidiol.

6. A composition according one of the preceding claims, wherein the extract of *Echinaceae purpurea L* is Echinacea dried press juice.

7. A composition according one of the preceding claims, wherein the extract of *Mangnolia officinalis* is Magnolia bark extract.

8. A composition according one of the preceding claims, wherein the method of treatment is a method of treating a dysregulated fatty acid profile.

9. A composition according one of the preceding claims, wherein the method of treatment comprises increasing the release of arachidonoyl ethanolamine by the microbiome of the patient.

10. A composition according one of the preceding claims, wherein the method of treatment comprises increasing the release of 2-Arachidonoylglycerol by the microbiome of the patient.
